# EUROPÄISCHE PATENTSCHRIFT

(11) **EP 0 867 195 B1**
(45) Veröffentlichungstag und Bekanntmachung des Hinweises auf die Patenterteilung: **22.01.2003**
(21) Anmeldenummer: 98103337.6
(22) Anmeldetag: 26.02.1998
(51) Int. Cl.: A61M 1/34, A61M 1/16

(54) **Vorrichtung zur Ultrafiltration bei der Hämodialyse**
Device for the ultrafiltration in a hemodialysis operation
Dispositif pour l'ultrafiltration pendant une hémodialyse

(30) Priorität: 01.03.1997 DE 19708391
(43) Veröffentlichungstag der Anmeldung: 30.09.1998
(73) Patentinhaber: Fresenius Medical Care Deutschland GmbH, 61352 Bad Homburg v.d.H. (DE)
(72) Erfinder: Wamsiedler, Ralf, 97453 Schonungen (DE); Walter, Raimund, 97525 Schwebheim (DE)
(74) Vertreter: Luderschmidt, Schüler & Partner GbR

(56) Entgegenhaltungen:
- EP-A- 0 513 672
- DE-C- 2 838 414
- DE-C- 3 837 498
- DE-C- 4 116 178

## Beschreibung

Die Erfindung betrifft eine Vorrichtung zur Ultrafiltration bei der Hämodialyse.

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Bei der Hämodialyse (HD) überwiegt der diffuse Stofftransport, während bei der Hämofiltration (HF) ein konvektiver Stofftransport über die Membran stattfindet. Eine Kombination aus beiden Verfahren ist die Hämodiafiltration (HDF).

Wegen der großen Austauschmengen besteht bei den genannten Verfahren die Notwendigkeit der exakten Bilanzierung von entzogener Flüssigkeit einerseits zur zugeführten Flüssigkeit andererseits und der zu ultrafiltrierenden Menge über die gesamte Behandlungszeit. Zum Stand der Technik gehören gravimetrische und volumetrische Bilanziersysteme.

Aus der DE-PS 28 38 414 ist eine Dialysevorrichtung bekannt, die eine volumetrische Bilanziereinrichtung aufweist. Die Bilanziereinrichtung besteht aus zwei durch je ein verschiebbares Element unterteilten Kammern, die je eine Zuführleitung für frische und eine mit einem Auslaß verbundene Abführleitung für verbrauchte Dialysierflüssigkeit aufweisen. In den Zuführ- und Abführleitungen sind Absperrventile angeordnet, die von einer Steuereinheit angesteuert und geschaltet werden. Zum Fördern der verbrauchten Dialysierflüssigkeit ist im Dialysierflüssigkeitsweg zwischen Dialysator und Bilanziereinrichtung eine Pumpe angeordnet. Weiterhin ist in der Zuführleitung zum Dialysator ein Dialysatorventil und in der Abführleitung des Dialysators ein Luftabscheider vorgesehen.

Die bekannte Vorrichtung wird derart betrieben, daß frische Dialysierflüssigkeit von einer Dialysierflüssigkeitsquelle den beiden Bilanzkammern abwechselnd durch entsprechende Schaltung der Absperrventile in den Zuführleitungen zugeführt wird. Gleichzeitig wird frische Dialysierflüssigkeit aus einem bereits gefüllten Raum der anderen Bilanzkammer dem Dialysator zugeführt, wo durch Diffüsion dem ebenfalls durch den Dialysator fließenden Blut die gewünschten Giftstoffe entzogen werden. Die verbrauchte Dialysierflüssigkeit wird anschließend in den zweiten Raum derselben Bilanzkammer gepumpt, von der die verbrauchte Dialysierflüssigkeit dann in den Auslaß geleitet wird.

Der zwischen der Bilanziereinrichtung und dem Dialysator eingeschlossene Teil des Flüssigkeitskreislaufes verhält sich wie ein geschlossenes, volumenkonstantes System. Um aus diesem System Flüssigkeit abzuführen, ist eine Entnahmevorrichtung vorgesehen.

Die mittels der Entnahmevorrichtung dem System entzogene Flüssigkeitsmenge wird aufgrund der erwähnten Eigenschaften der Bilanziereinrichtung durch eine gleich große Flüssigkeitsmenge ersetzt, die von der Blutseite zur Dialysierflüssigkeitsseite der Dialysatormembran übergeht. Die entzogene Flüssigkeitsmenge kann dem Patienten wieder als Substitutionsflüssigkeit zugeführt werden. Eine Substitution der entzogenen Flüssigkeitsmenge im Verhältnis 1:1 ist aber nicht zwingend erforderlich. Vielmehr kann es für die Behandlung in einzelnen Fällen von Vorteil sein, wenn eine größere Flüssigkeitsmenge entzogen wird als die Menge der Substitutionsflüssigkeit, die dem Patienten wieder zugeführt wird, d.h. trotz Substitution eine Netto-Ultrafiltration stattfindet.

An die Genauigkeit der Entnahmevorrichtung, die eine Steuerung der Ultrafiltration ermöglicht, sind hohe Anforderungen zu stellen. Um eine genau vorher bestimmbare Menge Ultrafiltrat dem Dialysierflüssigkeitsweg entziehen zu können, ist die Entnahmevorrichtung mit einer volumetrischen Membranpumpe ausgerüstet, wobei jeder einzelne Pumpenhub einer Einheitsmenge Ultrafiltrat entspricht. Nachteilig ist, daß zum Entziehen einer genau vorherbestimmbaren Menge Ultrafiltrat eine separate Pumpe mit einem relativ komplizierten Aufbau erforderlich ist.

Die DE 38 37 498 C2 beschreibt eine Vorrichtung, bei der eine Ultrafiltration ohne zusätzliche Ultrafiltratpumpe durchgeführt wird. Das Ultrafiltrat wird nicht über eine separate Entnahmevorrichtung im Dialysierflüssigkeitsweg zwischen Dialysatorausgang und Bilanziereinrichtung entnommen, sondern aus dem Dialysierflüssigkeitsweg über die Bilanziereinrichtung kontrolliert abgeführt, wobei zwischen zwei Hämodialysezyklen der Dialysierflüssigkeitsweg unterbrochen wird. Die Bilanziereinrichtung der bekannten Vorrichtung weist zwei Bilanzkammern auf. Während Ultrafiltrat in die eine Bilanzkammerhälfte der einen Bilanzkammer strömt, wird frische Dialysierflüssigkeit aus der anderen Bilanzkammerhälfte in eine der Bilanzkammerhälften der anderen Bilanzkammer umgeleitet.

Darüber hinaus ist eine Vorrichtung zur Ultrafiltration bei der Dialyse bekannt, bei der die Bestimmung der Ultrafiltratmenge mittels einer Bilanzkammer erfolgt. Die Bilanzkammer ist durch eine flexible Trennwand in zwei Kammerhälften unterteilt, die wechselseitig mit dem Ultrafiltrat befüllt werden, das dem Dialysierflüssigkeitsweg entzogen wird, wobei der Inhalt der jeweils anderen Kammerhälfte verworfen wird. Eine Bilanzierung von Ultrafiltrat gegen Substituat ist bei der bekannten Vorrichtung allerdings nicht möglich.

Eine Hämofiltrationsvorrichtung, bei der die Bilanzierung von Ultrafiltrat gegen Substituat mittels einer Bilanzkammer erfolgt, ist aus der DE 41 16 178 C1 bekannt.

Der Erfindung liegt die Aufgabe zugrunde, eine Vorrichtung zur Ultrafiltration bei der Hämodialyse anzugeben, die eine genaue Bilanzierung von Ultrafiltrat gegen Substituat erlaubt, ohne daß eine volumetrisch präzise arbeitende Ultrafiln-ationspumpe erforderlich ist.

Die Lösung dieser Aufgabe erfolgt erfindungsgemäß mit den im Patentanspruch 1 angegebenen Merkmalen.

Bei der erfindungsgemäßen Vorrichtung zur Ultrafiltration während der Hämodialyse wird die Ultrafiltrationsmenge mittels einer Bilanzkammer einer Ultrafiltrat-Bilanziereinrichtung bestimmt, die durch eine verschiebbare Membran in zwei Bilanzkammerhälften unterteilt ist. Eine volumetrisch präzise arbeitende Ultrafiltrationspumpe ist bei dem erfindungsgemäßen Verfahren nicht erforderlich.

Die beiden Bilanzkammerhälften der Bilanzkammer der Ultrafiltrat-Bilanziereinrichtung werden wechselweise mit verbrauchter Dialysierflüssigkeit und Substituat befüllt, wobei der Inhalt der jeweils anderen Kammerhälfte verworfen wird. In einem ersten Takt wird die eine Kammerhälfte mit verbrauchter Dialysierflüssigkeit, die dem Dialysierflüssigkeitsweg entzogen wird, unter Verwerfung von zuvor in die andere Kammer geleiteten Substituats befüllt. In einem zweiten Takt wird dann die andere Kammerhälfte mit Substituat unter Verwerfung der verbrauchten Dialysierflüssigkeit befüllt. Zur Bestimmung der Ultrafiltrationsmenge bzw. der Menge des Substituats wird das Füllvolumen der Bilanzkammer herangezogen.

Wenn das aus der einen Kammerhälfte verworfene Substituat dem Blutkreislauf während der gesamten Behandlungszeit zugeführt wird, findet eine 1:1 Bilanzierung statt, d.h. der Patient wird weder ultrafiltriert noch überwässert. Andererseits kann dem Dialysierflüssigkeitsweg aber auch mehr Ultrafiltrat entzogen werden, als dem Blutkreislauf Substituat zugeführt wird. In diesem Fall wird das aus der einen Kammerhälfte verworfene Substituat während der gesamten Behandlungszeit oder auch nur in einzelnen Zyklen nicht dem Blutkreislauf zugeführt, sondern wieder in die Substituatquelle zurückgeführt oder einfach in einen Auslaß abgeführt. Durch einen abwechselnden Betrieb in diesen beiden Modi ist eine genaue Steuerung der Netto-Ultrafiltrationsmenge über den gesamten Zeitraum der Dialyse möglich.

Die Substitionsflüssigkeit kann entweder separat zur Verfügung gestellt werden oder aus der Dialysierflüssigkeit gewonnen werden (Online-HDF). Vorzugsweise wird als Substituat frische Dialysierflüssigkeit in die andere Bilanzkammerhälfte der Bilanzkammer der Ultrafiltrat-Bilanziereinrichtung geleitet. Wenn die Substitutionsflüssigkeit nicht aus der Dialysierflüssigkeit gewonnen wird, ist eine externe Vorrichtung zur Bereitstellung der Substitutionsflüssigkeit erforderlich, die das Substituat mittels einer Pumpe oder durch Schwerkraft fördert. An eine derartige Pumpe sind aber im Hinblick auf die Fördergenauigkeit keine hohen Anforderungen zu stellen.

Die frische Dialysierflüssigkeit wird gegen die verbrauchte Dialysierflüssigkeit vorzugsweise in einer Dialysierflüssigkeits-Bilanziereinrichtung bilanziert, die zwei parallel geschaltete Bilanzkammern aufweist, die im Gegentakt arbeiten.

Von Vorteil ist, daß die Ansteuerung der Bilanzkammer der Ultrafiltrat-Bilanziereinrichtung über die Bilanzkammern der Dialysierflüssigkeits-Bilanziereinrichtung erfolgen kann. Die Ansteuerung der in den Zuführ- und Abführleitungen der Bilanzkammer der Ultrafiltrat-Bilanziereinrichtung angeordneten Absperrorgane erfolgt zweckmäßigerweise im gleichen Takt wie die Absperrorgane der Dialysierflüssigkeits-Bilanziereinrichtung.

Beispielhafte Ausführungsformen der Erfindung werden nachfolgend anhand der Zeichnungen näher erläutert.

Es zeigen:
- Fig. 1: eine vereinfachte schematische Darstellung einer bevorzugten Ausführungsform der Hämodiafiltrationsvorrichtung, wobei die Öffnungs- bzw. Schließstellung der Absperrorgane der Bilanzkammern im Ultrafiltrationsmodus während eines ersten Taktes dargestellt sind,
- Fig. 2: die Hämodiafiltrationsvorrichtung von Fig. 1 im Ultrafiltrationsmodus während eines zweiten Taktes,
- Fig. 3: die Hämodiafiltrationsvorrichtung von Fig. 1, wobei die Öffnungs- bzw. Schließstellung der Absperrorgane im Substitutionsmodus während eines ersten Taktes dargestellt sind und
- Fig. 4: die Hömodiafiltrationsvorrichtung von Fig. 1 im Substitutionsmodus während eines zweiten Taktes.

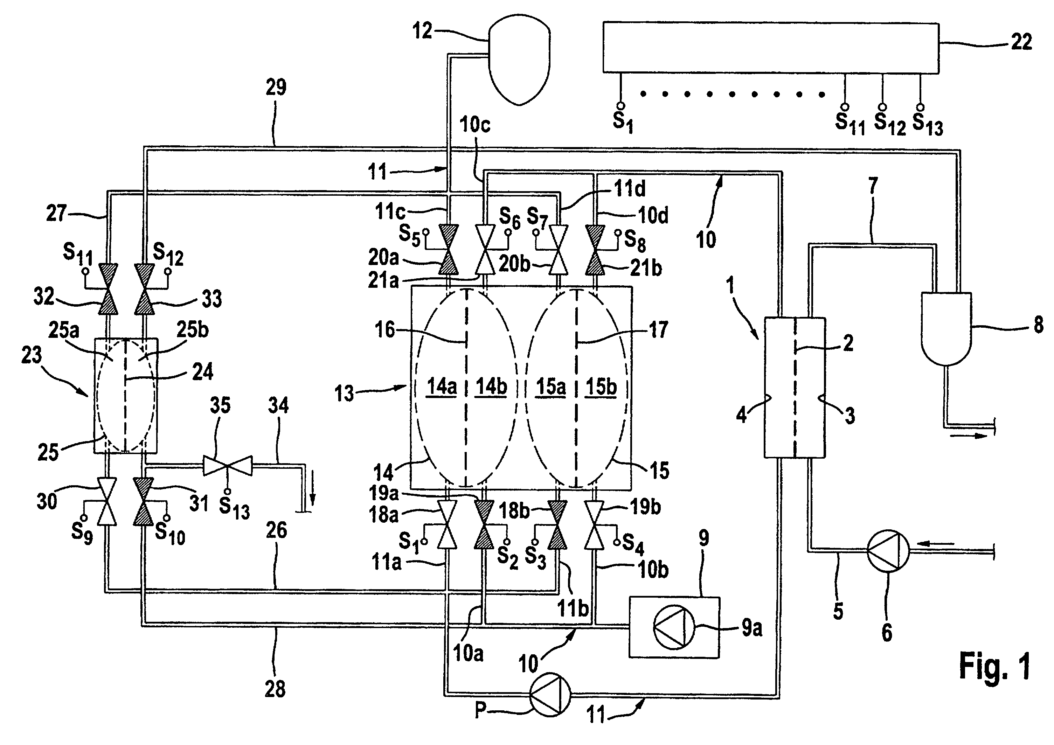

Fig. 1 zeigt eine bevorzugte Ausführungsform der Hämodiafiltrationsvorrichtung in vereinfachter schematischer Darstellung. Die Hämodiafiltrationsvorrichtung weist einen Dialysator 1 auf, der durch eine semipermeable Membran 2 in eine Blutkammer 3 und eine Dialysierflüssigkeitskammer 4 unterteilt ist. An dem Einlaß der Blutkammer 3 ist eine Blutzuführleitung 5 angeschlossen, in die eine Blutpumpe 6 geschaltet ist. Stromab der Blutkammer 3 führt eine Blutabflußleitung 7 von dem Auslaß der Blutkammer zu dem Patienten. In die Blutabflußleitung 7 ist eine Tropfkammer 8 geschaltet.

In einer Dialysierflüssigkeitsquelle 9, die über eine Entgasungspumpe 9a verfügt, wird frische Dialysierflüssigkeit bereitgestellt. Von der Dialysierflüssigkeitsquelle 9 führt eine Dialysierflüssigkeitszuleitung 10 zu dem Eingang der Dialysierflüssigkeitskammer 4 des Dialysators 1, während eine Dialysierflüssigkeitsableitung 11 von dem Ausgang der Dialysierflüssigkeitskammer 4 zu einem Abfluß 12 führt. Zur Bilanzierung der Dialysierflüssigkeit ist in der Dialysierflüssigkeitszuund -ableitung 10, 11 eine Dialysierflüssigkeits-Bilanziereinheit 13 geschaltet. Eine Dialysierflüssigkeitspumpe P ist stromab des Dialysators 4 in die Dialysierflüssigkeitszuleitung 10 geschaltet.

Die Dialysierflüssigkeits-Bilanziereinheit 13 weist zwei Bilanzkammern 14, 15 mit dem gleichen Füllvolumen auf, die jeweils durch eine bewegliche Trennwand 16, 17, z.B. in Form einer flexiblen Membran, in eine erste Bilanzkammerhälfte 14a bzw. 15a und eine zweite Bilanzkammerhälfte 14b bzw. 15b unterteilt sind. Die beiden Bilanzkammern 13, 14 haben in diesem Ausführungsbeispiel ein Füllvolumen von jeweils 30 ml.

Der zu der Dialysierflüssigkeits-Bilanziereinrichtung 13 führende Teil der Dialysierflüssigkeitszuleitung 10 teilt sich in zwei Leitungszweige 10a, 10b auf, von denen der eine Leitungszweig 10a zu dem Einlaß der zweiten Kammerhälfte 14b der ersten Bilanzkammer 14 und der andere Leitungsweg 10b zu dem Einlaß der zweiten Kammerhälfte 15b der zweiten Bilanzkammer 15 führt und der von der Bilanziereinrichtung 13 wegführende Teil der Dialysierflüssigkeitszuleitung 10 weist zwei Leitungszweige 10c, 10d auf, von denen der eine Leitungszweig 10c mit dem Auslaß der zweiten Kammerhälfte 14b der ersten Bilanzkammer 14 und der andere Leitungszweig 10d mit dem Auslaß der zweiten Kammerhälfte 15b der zweiten Bilanzkammer 15 verbunden ist.

Der zu der Dialysierflüssigkeits-Bilanziereinrichtung 13 führende Teil der Dialysierflüssigkeitsableitung 11 teilt sich ebenfalls in zwei Leitungsabschnitte 11a, 11b auf, von denen der eine Leitungsabschnitt 11a mit dem Einlaß der ersten Kammerhälfte 14a der ersten Bilanzkammer 14 und der andere Leitungsabschnitt 11b mit dem Einlaß der ersten Kammerhälfte 15a der zweiten Bilanzkammer 15 verbunden ist. Der Auslaß der ersten Kammerhälfte 14a der ersten Bilanzkammer 14 ist über den Leitungszweig 11c und der Auslaß der ersten Kammerhälfte 15a der zweiten Bilanzkammer 15 ist über den Leitungszweig 11d der Dialysierflüssigkeitsableitung 11 mit dem Abfluß 12 verbunden. In den einzelnen Leitungszweigen 10a bis 10d und 11a bis 11d sind Absperrorgane in Form von elektromagnetisch betätigbaren Ventilen 18a, 18b, 19a, 19b, 20a, 20b und 21a, 21b vorgesehen, die über Steuerleitungen S₁ bis S₈ an einer zentralen Steuereinheit 22 angeschlossen sind.

Neben der Dialysierflüssigkeits-Bilanziereinrichtung 13 umfaßt die Hämodiafiltrationsvorrichtung noch eine Ultrafiltrations-Bilanziereinrichtung 23, die eine durch eine bewegliche Trennwand 24, z.B. eine flexible Membran, in zwei Bilanzkammerhälften 25a, 25b unterteilte Bilanzkammer 25 aufweist. Diese Bilanzkammer 25 hat in dem Ausführungsbeispiel ein Füllvolumen von 6 ml.

Von dem zu der Dialysierflüssigkeits-Bilanziereinrichtung 13 führenden Teil der Dialysierflüssigkeitsableitung 11 zweigt stromauf der Ventile 18a, 18b eine Filtrateinlaßleitung 26 ab, die in den Einlaß der ersten Kammerhälfte 25a der Bilanzkammer 25 der Ultrafiltrations-Bilanziereinrichtung 23 mündet, während der Auslaß der ersten Kammerhälfte 25a über eine Filtratauslaßleitung 27 mit dem Abfluß 12 verbunden ist. Der Einlaß der zweiten Kammerhälfte 25b ist über eine Substituateinlaßleitung 28 mit der Dialysierflüssigkeitsquelle 9 stromauf der Ventile 19a, 19b verbunden. Von dem Auslaß der zweiten Kammerhälfte 25b führt eine Substituatauslaßleitung 29 in die in der Blutabführleitung 7 angeordnete Tropfkammer 8 (Post-Dilution). Alternativ kann die Zuführung des Substituats aber auch in eine stromauf des Dialysators angeordnete Tropfkammer erfolgen (Pre-Dilution).

In der Filtrateinlaß- und -auslaßleitung und der Substituateinlaß- und -auslaßleitung sind weitere Absperrorgane in Form von elektromagnetisch betätigbaren Ventilen 30, 31, 32, 33 angeordnet, die über weitere Steuerleitungen S₉ bis S₁₂ mit der zentralen Steuereinheit 22 verbunden sind. Von der Substituateinlaßleitung 28 zweigt stromab des Ventils 31 eine Ablaßleitung 34 ab, die zu der Dialysierflüssigkeitsquelle 9 führt. In der Ablaßleitung 34 ist ein weiteres Absperrorgan 35 angeordnet, das über eine weitere Steuerleitung S₁₃ an der zentralen Steuereinheit 22 angeschlossen ist.

Die erforderliche Sterilität der Dialysierflüssigkeit und insbesondere der Substitutionsflüssigkeit kann durch den Einbau von bekannten Sterilfiltern in das Flüssigkeitssystem gewährleistet werden, die der besseren Übersichtlichkeit halber nicht dargestellt sind.

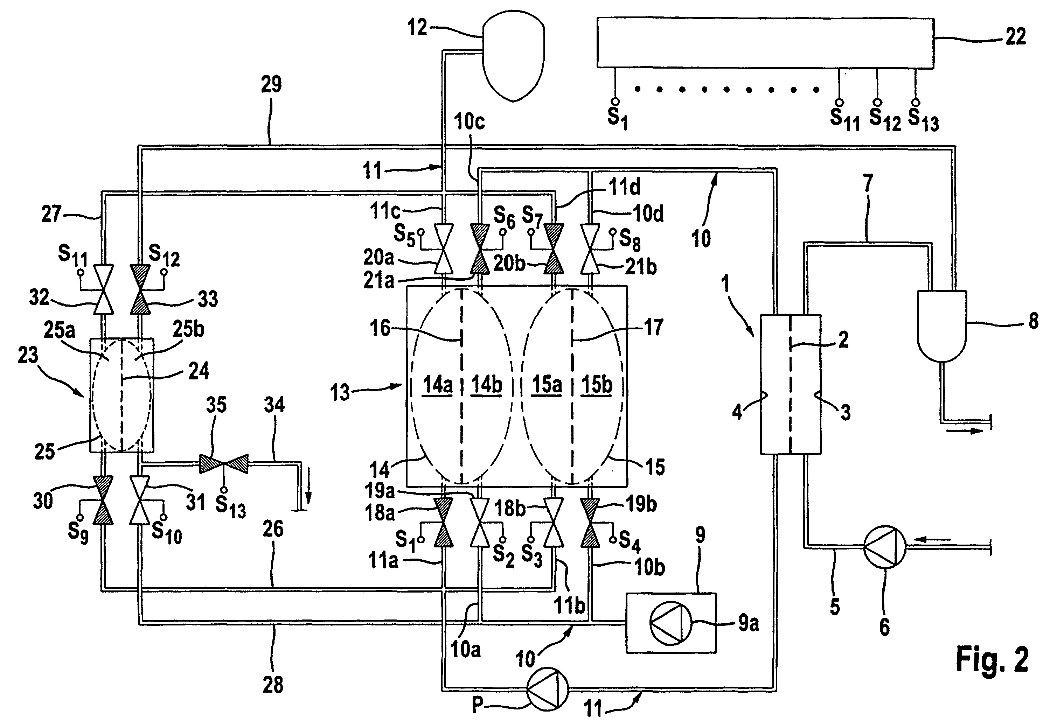

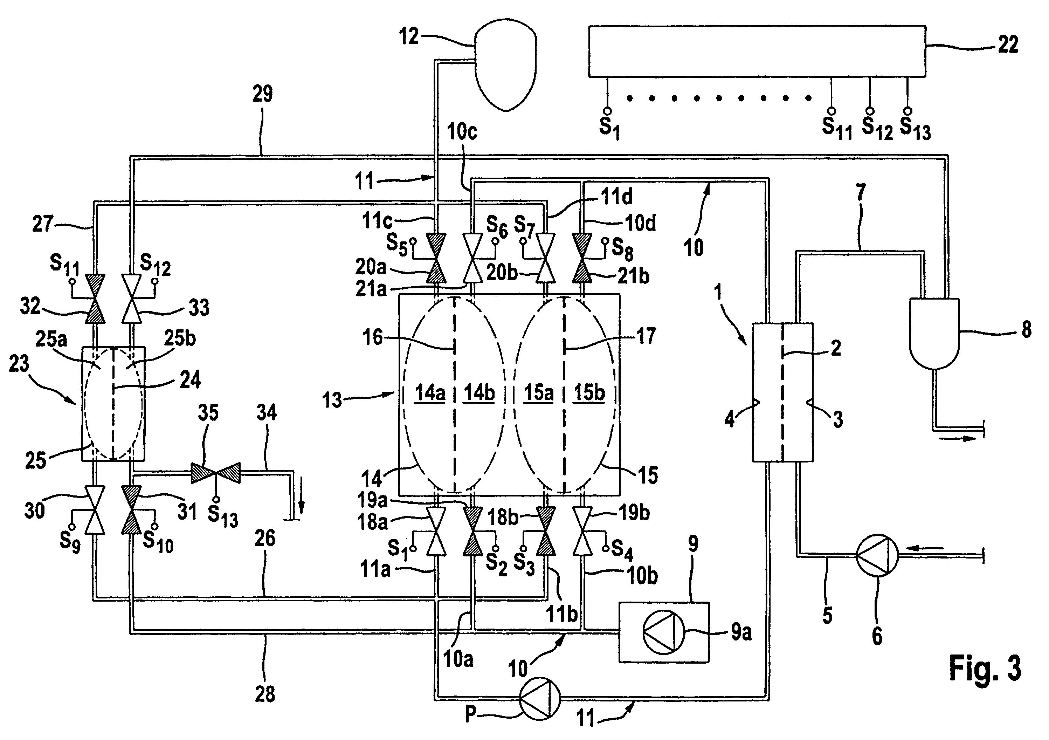

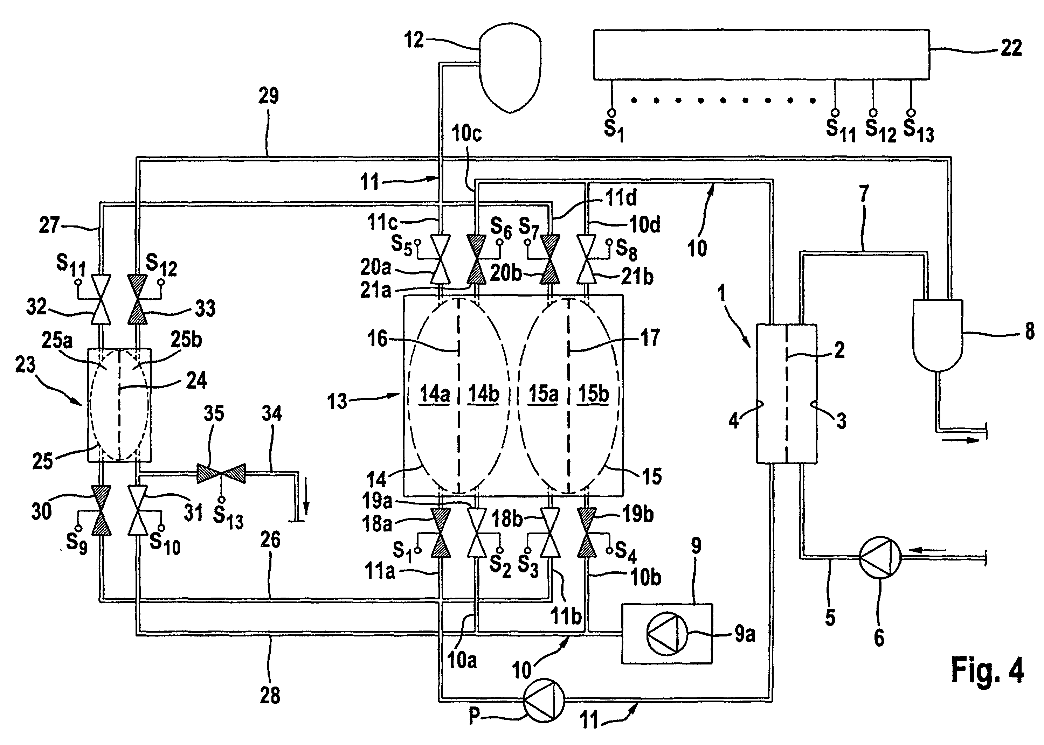

Die Fign. 1 und 2 zeigen die Schaltstellungen der Ventile, wenn die Hämodiafiltrationsvorrichtung im sogenannten Ultrafiltrationsmodus betrieben wird. Der Betrieb der Hämodiafiltrationsvorrichtung im sogenannten Substitutionsmodus zeigen die Fign. 3 und 4. Zur Verdeutlichung sind die geöffneten Ventile in den Figuren hell und die geschlossenen Ventile dunkel dargestellt.

Nachfolgend wird der Ultrafiltrationsmodus unter Bezugnahme auf die Fign. 1 und 2 im einzelnen beschrieben.

In einem ersten Takt werden die Ventile 18a, 19b, 21a, 20b, 30 und 35 von der zentralen Steuereinheit 22 geöffnet, wobei alle anderen Ventile geschlossen werden.

Frische Dialysierflüssigkeit (30 ml) strömt aus der Dialysierflüssigkeitsquelle 9 in die zweite Kammerhälfte 15b der zweiten Bilanzkammer 15, wodurch verbrauchte Dialysierflüssigkeit (30 ml), die in einem vorhergehenden Takt in die erste Kammerhälfte 15a geleitet wurde, in den Abfluß 12 verworfen wird. Gleichzeitig wird mittels der Dialysierflüssigkeitspumpe P verbrauchte Dialysierflüssigkeit (30 ml) aus der Dialysierflüssigkeitskammer 4 des Dialysators 1 in die erste Kammerhälfte 14a der ersten Bilanzkammer 14 gefördert, wodurch zuvor in die zweite Kammerhälfte 14b geleitete frische Dialysierflüssigkeit aus der zweiten Kammerhälfte verworfen und der Dialysierflüssigkeitskammer 4 zugeführt wird.

Während die Bilanzkammern 14 und 15 der Dialysierflüssigkeits-Bilanziereinrichtung 13 befüllt bzw. entleert werden, wird die erste Kammerhälfte 25a der Bilanzkammer 25 der Ultrafiltrations-Bilanziereinrichtung 23 mit verbrauchter Dialysierflüssigkeit (6 ml) befüllt, wodurch frische Dialysierflüssigkeit, die in einem vorhergehenden Takt in die zweite Kammerhälfte 25b geleitet wurde, bei geöffnetem Ventil 35 über die Abflußleitung 34 in die Dialysierflüssigkeitsquelle 9 zurückfließt. Die Abflußleitung 34 kann aber auch direkt mit dem Abfluß 12 verbunden sein.

In einem zweiten Arbeitstakt werden die Ventile 19a, 18b, 20a, 21b, 31 und 32 geöffnet, wobei alle anderen Ventil geschlossen werden. Frische Dialysierflüssigkeit strömt in die zweite Kammerhälfte 14b der ersten Bilanzkammer 14 der Dialysierflüssigkeits-Bilanziereinrichtung 13, wodurch verbrauchte Dialyierflüssigkeit aus der ersten Kammerhälfte 14a in den Abfluß 12 verworfen wird. Gleichzeitig wird verbrauchte Dialysierflüssigkeit in die erste Kammerhälfte 15a der zweiten Bilanzkammer 15 gefördert, wodurch frische Dialysierflüssigkeit aus der zweiten Kammerhälfte 15b verworfen und der Dialysierflüssigkeitskammer 4 zugeführt wird.

Während die Bilanzkammern 14 und 15 der Dialysierflüssigkeits-Bilanziereinrichtung 12 befüllt bzw. entleert werden, wird frische Dialysierflüssigkeit (6 ml) in die zweite Kammerhälfte 25b der Bilanzkammer 25 der Ultrafiltrations-Bilanziereinrichtung 23 geleitet, wodurch verbrauchte Dialysierflüssigkeit aus der ersten Kammerhälfte 25a in den Abfluß 12 verworfen wird. Daraufhin werden wieder die Ventile 18a, 19b, 21a, 20b, 30 und 35 geöffnet, wobei alle anderen Ventile geschlossen werden, so daß sich wieder der erste Takt anschließen kann (Fig. 1).

Der Flüssigkeitsentzug findet bei jedem zweiten Takt statt, wobei 3 ml Ultrafiltrat pro Bilanzkammertakt dem Dialysierflüssigkeitsweg entzogen wird, was 1/10 des Dialysatflusses entspricht.

Nachfolgend wird der Substitutionsmodus unter Bezugnahme auf die Fign. 3 und 4 im einzelnen beschrieben. Der Substitutionsmodus unterscheidet sich von dem Ultrafiltrationsmodus dadurch, daß das Substituat, d.h. die aus der zweiten Kammerhälfte 25b der Bilanzkammer 23 verworfene frische Dialysierflüssigkeit, nicht zu der Dialysierflüssigkeitsquelle 9 geleitet wird, sondern der Tropfkammer 8 zugeführt wird.

Fig. 3 zeigt die Schaltstellung der Ventile im ersten Takt, der dem ersten Takt im Ultrafiltrationsmodus (Fig. 1) mit der Ausnahme entspricht, daß das in der Substituatablaßleitung 29 angeordnete Ventil 33 nicht geschlossen, sondern geöffnet und das in der Ablaßleitung 34 angeordnete Ventil 35 nicht geöffnet, sondern geschlossen ist, so daß die aus der zweiten Kammerhälfte 25b verworfene frische Dialysierflüssigkeit als Substituat dem Patienten wieder durch die Abflußleitung 29, die Tropfkammer 8 und die Blutabflußleitung 7 zugeführt werden kann. Der zweite Takt im Substitutionsmodus ist mit dem zweiten Takt des Ultrafiltrationsmodus identisch. An den zweiten Zyklus schließt sich entsprechend dem Ultrafiltrationsmodus wieder der erste Zyklus an (Fig. 3).

Die Hämodiafiltrationsvorrichtung kann auch mit abgeschalteter Ultrafiltrations-Bilanziereinrichtung 23 betrieben werden, so daß es in den entsprechenden Zyklen zu keiner Ultrafiltration mehr kommt. Hierzu werden in einzelnen Zyklen oder über die gesamte Behandlungsdauer die Ventile 30, 31, 32 und 33 geschlossen.

## Patentansprüche

1. Vorrichtung zur Ultrafiltration bei der Hämodialyse mit einem durch eine semipermeable Membran in eine Blutkammer (3) und eine Dialysierflüssigkeitskammer (4) unterteilten Dialysator (1), wobei die Blutkammer Teil eines extrakorporalen Blutkreislaufs ist,
einer Dialysierflüssigkeitsquelle,
Zuführ- und Abführleitungen (10, 11) zum Zuführen von frischer Dialysierflüssigkeit aus der Dialysierflüssigkeitsquelle in die Dialysierflüssigkeitskammer des Dialysators und zum Abführen von verbrauchter Dialysierflüssigkeit aus der Dialysierflüssigkeitskammer in einen Auslaß (12),
einer in die Zuführ- und Abführleitungen geschalteten Dialysierflüssigkeits-Bilanziereinrichtung (13) zum Bilanzieren von frischer und verbrauchter Dialysierflüssigkeit, die mindestens eine durch eine bewegliche Trennwand (16) in zwei Bilanzkammerhälften (14a, 14b) unterteilte Bilanzkammer (14) mit in den Zuführ- und Abführleitungen der Bilanzkammerhälften angeordneten Absperrorganen (18a, 19a, 20a, 21a) aufweist,
einer an die Absperrorgane der Dialysierflüssigkeits-Bilanziereinrichtung angeschlossenen Steuereinheit (22),
einer Einrichtung zum Entziehen von Ultrafiltrat aus dem Dialysierflüssigkeitsweg und
einer Substituatquelle zum Zuführen von Substituat,
**dadurch gekennzeichnet,**
**daß** die Einrichtung zum Entziehen von Ultrafiltrat eine Ultrafiltrat-Bilanziereinrichtung (23) mit einer durch eine bewegliche Trennwand in zwei Bilanzkammerhälften (25a, 25b) unterteilten Bilanzkammer (25) aufweist, wobei die eine Kammerhälfte (25a) über eine Filtrateinlaßleitung (26) mit dem Dialysierflüssigkeitsweg und über eine Filtratauslaßleitung (27) mit dem Auslaß (12) in Verbindung steht und die andere Kammerhälfte (25b) der Bilanzkammer der Ultrafiltrat-Bilanziereinrichtung über eine Substituateinlaßleitung (28) mit der Substituatquelle in Verbindung steht und an die eine Substituatauslaßleitung (29) angeschlossen ist,
**daß** in den Filtrat- und Substituatleitungen an die Steuereinheit angeschlossene Absperrorgane (30, 31, 32, 33) angeordnet sind und
**daß** die Steuereinheit (22) derart ausgebildet ist, daß in einem ersten Takt eine Bilanzkammerhälfte (25a) der Bilanzkammer der Ultrafiltrat-Bilanziereinrichtung unter Verwerfung von Substituat aus der anderen Bilanzkammerhälfte mit verbrauchter Dialysierflüssigkeit befüllbar und in einem zweiten Takt die andere Bilanzkammerhälfte (25b) unter Verwerfung der verbrauchten Dialysierflüssigkeit aus der einen Bilanzkammerhälfte mit Substituat befüllbar ist.

2. Vorrichtung nach Anspruch 1, **dadurch gekennzeichnet, daß** die Sub tituatauslaßleitung (29) mit dem extrakorporalen Blutkreislauf in Ver indung steht.

3. Vorrichtung nach Anspruch 1 oder 2, **dadurch gekennzeichnet, daß die** Substituatquelle und die Dialysierflüssigkeitsquelle durch eine gemeinsame Versorgungseinheit (9) zur Bereitstellung frischer Dialysierflüssigkeit gebildet werden.

4. Vorrichtung nach einem der Ansprüche 1 bis 3, **dadurch gekennzeichnet, daß** im Dialysierflüssigkeitsweg eine Pumpe (P) zum Fördern der verbrauchten Dialysierflüssigkeit angeordnet ist.

5. Vorrichtung nach einem der Ansprüche 1 bis 4, **dadurch gekennzeichnet, daß** die Dialysierflüssigkeits-Bilanziereinrichtung (13) zwei Bilanzkammern (14, 15) aufweist, wobei die Bilanzkammern derart parallel geschaltet sind und die Steuereinheit (22) derart ausgebildet ist, daß in dem ersten Takt eine Bilanzkammerhälfte der einen Bilanzkammer mit verbrauchter Dialysierflüssigkeit unter Verwerfung von frischer Dialysierflüssigkeit aus der anderen Kammerhälfte in die Dialysierflüssigkeitskammer des Dialysators befüllbar ist und eine Bilanzkammerhälfte der zweiten Bilanzkammer mit frischer Dialysierflüssigkeit unter Verwerfung von verbrauchter Dialysierflüssigkeit aus der anderen Bilanzkammerhälfte in den Auslaß befüllbar ist und in dem zweiten Takt die andere Bilanzkammerhälfte der ersten Bilanzkammer mit frischer Dialysierflüssigkeit unter Verwerfung der verbrauchten Dialysierflüssigkeit aus der einen Bilanzkammerhälfte in den Auslaß befüllbar ist und die andere Bilanzkammerhälfte der zweiten Bilanzkammer mit verbrauchter Dialysierflüssigkeit unter Verwerfung der frischen Dialysierflüssigkeit aus der einen Bilanzkammerhälfte in die Dialysierflüssigkeitskammer des Dialysators befüllbar ist.

6. Vorrichtung nach einem der Ansprüche 1 bis 5, **dadurch gekennzeichnet, daß** das Füllvolumen der Bilanzkammer (25) der Ultrafiltrat-Bilanziereinrichtung (23) kleiner als das Füllvolumen der Bilanzkammer (14, 15) der Dialysierflüssigkeits-Bilanziereinrichtung (13) ist.

## Claims

1. Device for ultrafiltration in the course of dialysis of blood with a dialyser (1) divided by a semi-permeable membrane into a blood chamber (3) and a dialysing liquid chamber (4), whereby the blood chamber element forms part of an extra-corporal blood circulation circuit,
a source of dialysing liquid,
supply and discharge tubing (10, 11) to feed fresh dialysing liquid from the source of dialysing liquid into the dialysing liquid chamber of the dialyser and to convey the used dialysing liquid away from the dialysing liquid chamber to a discharge point (12),
a dialysing liquid balance device (13) located in the supply and discharge tube system of the dialyser to maintain a balance of fresh and used dialysing liquid which exhibits at least one balance chamber (14) divided by a moveable separating wall (16) into two balance chamber halves (14a, 14b) with closure fitments (18a, 19a, 20a, 21a) located in the supply and discharge tubing of the balance chamber halves,
a control unit (22) connected to the closure fitments of the dialysing liquid balance chamber device,
a device for withdrawing the ultrafiltrate from the dialysing liquid circulation path and a substituent source for supplying substituent material
**characterised in that** the device for withdrawing the ultrafiltrate exhibits an ultra-filtrate balancing device (23) with a balance chamber (25) divided into two balance chamber halves (25a, 25b) by a movable separating wall, whereby one chamber half (25a) is connected to the dialysing liquid circulation path by means of a filtrate supply tube (26) and is connected to the discharge point (12) by a filtrate discharge tube (27) while the other chamber half (25b) of the balance chamber of the ultra-filtrate balancing device is connected by a substituent material supply tube (28) to the substituent material source and to which is attached a substituent material discharge tube (29),
that closure fitments (30, 31, 32 and 33) are provided in the filtrate and substituent material tubes, which are connected to the control unit and
that the control unit (22) is designed in such a manner that in a first cycle a balance chamber half (25a) of the balance chamber in the ultrafiltrate balancing device is capable of being filled with used dialysis liquid while discharging substituent material from the other balance chamber half and in a second cycle the other balance chamber half 25b is capable of being filled with substituent material while discharging the used dialysing liquid from the first balance chamber half.

2. Device in accordance with claim 1, **characterised in that** the substituent material discharge tube (29) is connected to the extra-corporal blood circulation system.

3. Device in accordance with claim 1 or claim 2, **characterised in that** the source of substituent material and the source of the dialysing liquid are formed by a common supply unit (9) for the supply of fresh dialysing liquid.

4. Device in accordance with one of claims 1 to 3, **characterised in that** a pump (P) is provided in the dialysing liquid circulation path to propel the used dialysing liquid.

5. Device in accordance with one of claims 1 to 4, **characterised in that** the dialysing liquid balancing device (13) exhibits two balancing chambers (14, 15) where the balance chambers are so arranged in parallel and the control unit (22) is of such a design that in the first cycle one balance-chamber half of the one balance chamber is capable of being filled with used dialysing liquid while fresh dialysis liquid is conveyed from the other chamber half into the dialysis liquid chamber of the dialyser and one balance chamber half of the second balance chamber is capable of being filled with fresh dialysing liquid while used dialysing liquid from the other balance chamber half is conveyed to the discharge point, and in the second cycle, the other balance chamber half of the first balance chamber is capable of being filled with fresh dialysing liquid while the used dialysing liquid from the first balance chamber half is conveyed to the discharge point and the other balance chamber half of the second balance chamber is capable of being filled with used dialysing liquid while fresh dialysing liquid is conveyed out of the first balance chamber half into the dialysing liquid chamber of the dialyser.

6. Device in accordance with one of the claims 1 to 5, **characterised in that** the capacity of the balance chamber (25) of the ultrafiltrate balancing device (23) is smaller than the capacity of the balance chamber (14, 15) of the dialysing liquid balance device (13).

## Revendications

1. Dispositif d'ultrafiltration pour l'hémodialyse avec un dialyseur (1) partagé par une membrane semi-perméable en une chambre sanguine (3) et une chambre de liquide de dialyse (4), la chambre sanguine faisant partie d'une circulation sanguine extracorporelle,
une source de liquide de dialyse,
des conduites d'alimentation et d'évacuation (10,11) pour l'alimentation en liquide de dialyse frais provenant de la source de liquide de dialyse dans la chambre de liquide de dialyse du dialyseur et pour l'évacuation de liquide de dialyse usé provenant de la chambre de liquide de dialyse vers une ouverture de sortie (12),
un dispositif d'établissement du bilan du liquide de dialyse (13) branché sur les conduites d'alimentation et d'évacuation, pour l'établissement du bilan du liquide de dialyse frais et usé, comprenant une chambre de bilan (14) partagée par une cloison mobile (16) en deux demi-chambres de bilan (14a,14b) avec des organes d'obturation (18a,19a,20a,21a) disposés dans les conduites d'alimentation et d'évacuation des demi-chambres de bilan,
une unité de commande (22) branchée sur les organes d'obturation du dispositif d'établissement du bilan du liquide de dialyse,
un dispositif d'élimination de l'ultrafiltrat du trajet du liquide de dialyse et
une source de substitut pour l'alimentation en substitut,
**caractérisé en ce que**
le dispositif d'élimination de l'ultrafiltrat comporte un dispositif d'établissement de bilan de l'ultrafiltrat (23) avec une chambre de bilan (25) partagée en deux demi-chambres de bilan (25a,25b) par une cloison mobile, une des demi-chambres (25a) étant reliée au trajet du liquide de dialyse par l'intermédiaire d'une conduite d'entrée du filtrat (26) et à l'ouverture de sortie (12) par l'intermédiaire d'une conduite d'évacuation du filtrat (27) et l'autre demi-chambre (25b) de la chambre de bilan du dispositif d'établissement de bilan de l'ultrafiltrat étant reliée à la source de substitut par l'intermédiaire d'une conduite d'alimentation en substitut (28) et à laquelle une conduite d'évacuation du substitut (29) est branchée,
**en ce que**, dans les conduites de filtrat et de substitut, sont disposés des organes d'obturation (30,31,32,33) branchés à l'unité de commande et
**en ce que** l'unité de commande (22) est configurée de telle sorte que, dans un premier temps, une première demi-chambre de bilan (25a) de la chambre de bilan du dispositif de bilan de l'ultrafiltrat peut être remplie de liquide de dialyse usé avec éjection du substitut de l'autre demi-chambre de bilan et, dans un deuxième temps, l'autre demi-chambre de bilan (25b) peut être remplie de substitut avec éjection du liquide de dialyse usé de la première demi-chambre de bilan.

2. Dispositif selon la revendication 1, **caractérisé en ce que** la conduite d'évacuation du substitut (29) est reliée à la circulation sanguine extracorporelle.

3. Dispositif selon la revendication 1 ou 2, **caractérisé en ce que** source de substitut et la source de liquide de dialyse sont constituées d'une unité d'alimentation commune (9) pour la production de liquide de dialyse frais.

4. Dispositif selon l'une des revendications 1 à 3, **caractérisé en ce que**, dans le trajet du liquide de dialyse, est disposée une pompe (P) pour le refoulement du liquide de dialyse usé.

5. Dispositif selon l'une des revendications 1 à 4, **caractérisé en ce que** le dispositif d'établissement du bilan du liquide de dialyse (13) comporte deux chambres de bilan (14,15), les chambres de bilan étant branchées en parallèle et l'unité de commande (22) étant configurée de telle sorte que, dans un premier temps, une première demi-chambre de bilan d'une première des chambres de bilan peut être remplie de liquide de dialyse usé avec éjection du liquide de dialyse frais de l'autre demi-chambre vers la chambre de liquide de dialyse du dialyseur et une première demi-chambre de bilan de la deuxième chambre de bilan peut être remplie de liquide de dialyse frais avec éjection du liquide de dialyse usé de l'autre demi-chambre de bilan vers l'ouverture de sortie et, dans un deuxième temps, l'autre demi-chambre de bilan de la première chambre de bilan peut être remplie de liquide de dialyse frais avec éjection du liquide de dialyse usé de la première demi-chambre de bilan et l'autre demi-chambre de bilan de la deuxième chambre de bilan peut être remplie de liquide de dialyse usé avec éjection du liquide de dialyse frais de la première demi-chambre de bilan vers la chambre de liquide de dialyse du dialyseur.

6. Dispositif selon l'une des revendications 1 à 5, **caractérisé en ce que** le volume de remplissage de la chambre de bilan (25) du dispositif d'établissement du bilan de l'ultrafiltrat (23) est inférieur au volume de remplissage de la chambre de bilan (14,15) du dispositif d'établissement du bilan du liquide de dialyse (13).
